# DEMANDE DE BREVET EUROPEEN

(11) **EP 1 779 889 A1**
(43) Date de publication de la demande: **02.05.2007**
(21) Numéro de dépôt: 05110208.5
(22) Date de dépôt: 31.10.2005
(51) Int. Cl.: A61N 1/05, A61N 1/375

(54) **Sonde de stimulation cardiaque bipolaire à pole auxiliaire**

(71) Demandeur: Bemurat, Marc, F-33850 Leognan (FR); Bemurat, Laurent, F-33850 Leognan (FR); Bemurat, Eliane, F-33850 Leognan (FR); Bemurat, Diane, F-33850 Leognan (FR)
(72) Inventeur: Bemurat, Marc, F-33850 Leognan (FR); Bemurat, Laurent, F-33850 Leognan (FR); Bemurat, Eliane, F-33850 Leognan (FR); Bemurat, Diane, F-33850 Leognan (FR)
(74) Mandataire: Schmit, Christian Norbert Marie

(57) **Abrégé**

L'objet de l'invention est une sonde de stimulation cardiaque bipolaire à conducteurs à enroulements colinéaires et à pôle auxiliaire, caractérisée en ce que ledit pôle auxiliaire (P_{A}) est relié à l'un des deux connecteurs (D,P) de liaison de la sonde (1) au boîtier de stimulateur par un troisième conducteur à enroulement colinéaire avec les deux conducteurs de la sonde sur un tronçon commun à trois conducteurs entre l'un desdits connecteurs (D,P) et une sortie de dérivation (A,A').

## Description

La présente invention se rapporte aux sondes de stimulation cardiaque bipolaires et à pôle auxiliaire de stimulation temporaire externe permettant de procéder au remplacement du boîtier du stimulateur sans cesser pour autant de stimuler le coeur.

L'invention vise à simplifier la conception de ce type connu de sonde et à rendre plus facile et efficace l'accès au pôle auxiliaire de stimulation.

A cet effet, l'invention a pour objet une sonde de stimulation cardiaque bipolaire à conducteurs à enroulements colinéaires et à pôle auxiliaire, caractérisée en ce que ledit pôle auxiliaire est relié à l'un des deux connecteurs de liaison de la sonde au boîtier de stimulateur par un troisième conducteur à enroulement colinéaires avec les deux conducteurs de la sonde.

Un tel agencement colinéaire des trois conducteurs de la sonde solutionne de manière élégante la disposition de ces trois conducteurs et leurs connexions, notamment pour ce qui concerne le conducteur affecté au pôle auxiliaire de stimulation qui est en dérivation en un endroit de la sonde le plus approprié, par exemple à la jonction entre le cordon de la sonde et l'embout de connection au boîtier de stimulation.

La sonde conserve un diamètre réduit du fait de la colinéarité des trois conducteurs sur la totalité du trajet commun et présente une grande fiabilité.

Le conducteur auxiliaire est de préférence connecté au connecteur de sonde relié à l'électrode de stimulation distale, mais il pourrait l'être à l'autre connecteur, relié à l'électrode proximale.

L'invention concerne également des structures de pôle auxiliaire de stimulation appropriées à ce nouveau type de sonde.

D'autres caractéristiques et avantages ressortiront de la description qui va suivre de modes de réalisation de l'invention, description donnée à titre d'exemple uniquement et en regard des dessins annexés sur lesquels :
- Figure 1 est en schéma illustrant le principe de réalisation de la sonde de l'invention ;
- Figure 2 illustre le mode de connexion au connecteur de l'électrode distale du conducteur du pôle auxiliaire et de l'un des deux conducteurs de la sonde ;
- Figure 3 illustre une variante de connexion du conducteur du pôle auxiliaire ;
- Figure 4 représente la zone de dérivation de la sonde avec son pôle auxiliaire ;
- Figure 5 illustre le mode d'utilisation de la sonde ;
- Figure 6 représente un premier mode de réalisation du pôle auxiliaire,
- Figures 7 et 8 représentent un second mode de réalisation du pôle auxiliaire,
- Figure 9 représente une variante de positionnement de la sortie du pôle auxiliaire.

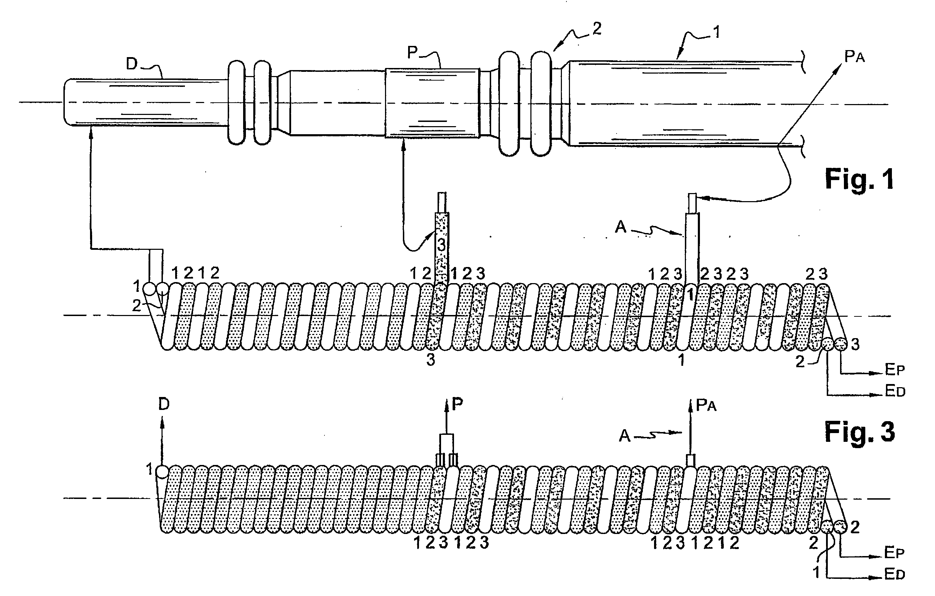

Sur la figure 1, on a représenté partiellement en 1 une sonde bipolaire selon l'invention avec son embout 2 de connexion à un boîtier de stimulateur (non représenté) faisant apparaître un premier connecteur D de liaison du stimulateur à l'électrode distale E_{D} de la sonde et un second connecteur P de liaison à l'électrode proximale E_{P}.

Les deux conducteurs de la sonde 1 sont conventionnels et du type colinéaire, à savoir deux conducteurs à enroulements à spires jointives imbriqués de même diamètre.

Les conducteurs sont à cet effet constitués d'une âme centrale conductrice enveloppées dans une gaine isolante, la colinéarité permettant de réduire le diamètre de la sonde 1 de manière substantielle, par exemple 1,7 mm au lieu de 2,3 mm pour des sondes traditionnelles à deux conducteurs spiralés coaxiaux de diamètres différents.

Sur la figure 1, on a représenté les trois conducteurs colinéaires identifiés respectivement par les repères 1, 2 et 3. Le conducteur N° 1 est le conducteur du pôle auxiliaire et donc relié électriquement au connecteur D dans le mode de réalisation de la figure 1. L'autre extrémité du conducteur N°1 est sortie de l'enroulement commun et isolée en A, cette extrémité A étant reliée électriquement à un pôle auxiliaire P_{A} (non représenté sur la figure 1).

Le conducteur N°2 est également relié électriquement au connecteur D, cependant que son autre extrémité sera connectée à l'électrode distale E_{D} comme illustré par la figure 1.

Le conducteur N°3 est le conducteur de sonde reliant le connecteur P à l'électrode proximale E_{P}.

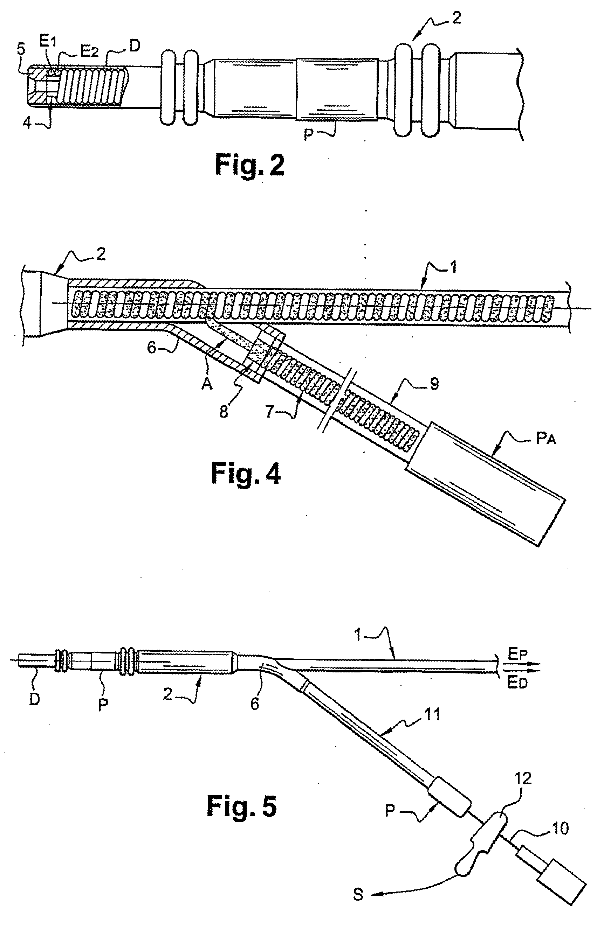

La figure 2 illustre un mode de connexion électrique entre le connecteur D constitué d'une manchon métallique tubulaire à l'intérieur duquel sont disposés les deux conducteurs colinéaires N° 1 et 2 dont les extrémités E₁ et E₂, sont soudées à un manchon épaulé 4 engagé dans l'espace interne aux conducteurs et dont la tête 5 ferme l'extrémité du manchon D, la tête étant soudée au laser au manchon.

La figure 3 illustre une variante de connexion du conducteur auxiliaire (N°3) relié, à une extrémité, au connecteur P et, à l'autre extrémité, au pôle auxiliaire P_{A}.

Le connecteur D est toujours relié à l'électrode distale E_{D} via le conducteur N°1 et le conducteur P est relié à l'électrode proximale E_{P} via le conducteur N°2.

Ainsi, les enroulements colinéaires ont, dans les deux modes de connexion des figures 1 et 3, un tronçon commun à trois conducteurs entre le connecteur P et le point de sortie A du conducteur de dérivation et un tronçon commun à deux conducteurs entre ladite sortie A et les électrodes de la sonde. Par contre, dans la zone entre les deux connecteurs D et P, il y a deux conducteurs (N° 1 et 2) dans la figure 1 pour un seul conducteur (N°1) dans la figure 3.

La figure 4 représente la dérivation de la sonde 1 acheminant le conducteur de dérivation (sortie A) au pôle auxiliaire de stimulation P_{A}. La dérivation est réalisée à la jonction du cordon de la sonde à l'embout 2 de connection au boîtier et se matérialise par un manchon 6 en matériau isolant tel que du silicone.

La liaison électrique entre l'extrémité A du conducteur auxiliaire et le pôle P_{A} est réalisée par un conducteur spirale dénudé 7, relié à l'extrémité A par un bouchon conducteur 8 de liaison et enveloppé par une gaine isolante 9 en silicone.

La figure 5 illustre le mode d'emploi du pôle auxiliaire P_{A}. Un stylet conducteur 10 est inséré à l'intérieur du pôle P_{A} en sorte de venir en contact avec le conducteur 7 de la dérivation 11. Une pince crocodile 12 par exemple est appliquée sur le stylet 10 et reliée électriquement à un stimulateur externe 5 (non représenté).

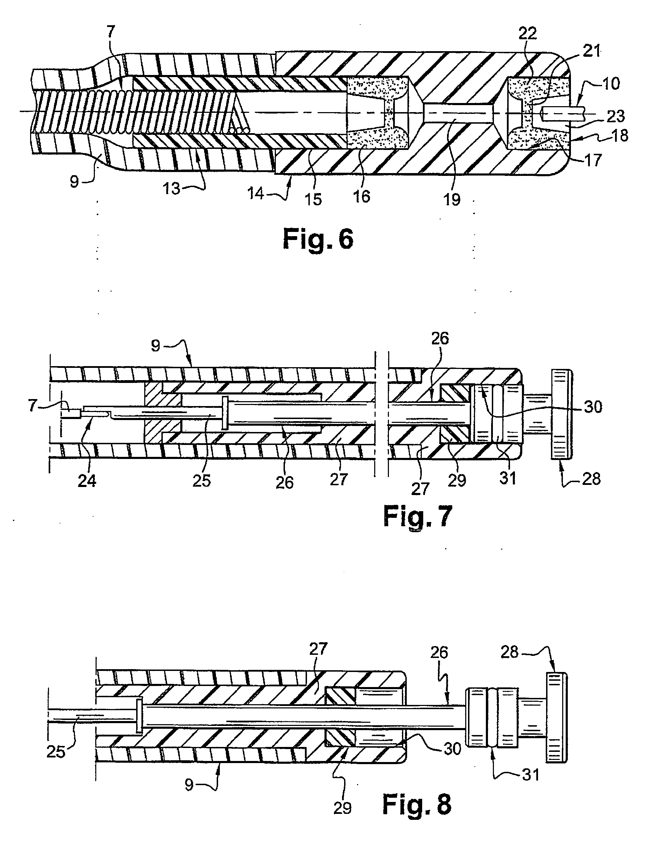

La figure 6 représente un premier mode de réalisation d'un pôle auxiliaire P_{A}. L'extrémité du conducteur 7 est insérée à force au moins partiellement dans un manchon 13 en matériau isolant électrique, lui-même emmanché partiellement dans un second manchon 14 en matériau isolant électrique.

Le manchon 14 comporte, à une extrémité, un logement cylindrique 15 en partie occupé par le manchon 13, le reste du logement 15 étant occupé par un obturateur 16 en silicone ou analogue. L'autre extrémité du manchon 15 définit un logement cylindrique 17 recevant un obturateur 18 en silicone ou analogue identique à l'obturateur 16.

Entre les deux logements 15, 17 est ménagé coaxialement à ces derniers, un conduit 19 de diamètre réduit.

Les obturateurs 16,18 ont une forme qui facilite l'insertion du stylet 10 de la figure 5.

L'obturateur d'entrée 18 présente un voile mince central 21 entouré d'une partie annulaire 22 plus massive définissant une cavité tronconique 23 de guidage du stylet 10 en direction du voile central 21 de diamètre légèrement supérieur à celui du canal de guidage 19. Le voile 21 peut être pré-percé ou pré-fendu.

Ainsi, la pénétration du stylet 10 dans l'embout de raccordement 14 est parfaitement guidée et l'extrémité du stylet est parfaitement centrée sur l'axe du conducteur 7.

Le stylet 10 est engagé à l'intérieur du conducteur 7 sur une certaine distance et parfaitement maintenu immobile par rapport à l'embout par la pression de la matière des deux obturateurs 16, 18 traversés. On a ainsi l'assurance d'avoir un contact physique entre le stylet et le conducteur fiable et efficace électriquement parlant, tout en ayant au niveau de l'embout 14 une étanchéité optimale grâce au double obturateur (16, 18).

Les figures 7 et 8 représentent un second mode de réalisation du pôle auxiliaire P_{A}.

En 24, est représentée l'extrémité (côté pôle auxiliaire) du conducteur 7 de dérivation.

Cette extrémité 24 est soudée à un fil en acier inoxydable 25 sur lequel peut coulisser un tube 26 également en acier inoxydable, monté lui-même coulissant dans un manchon isolant 27 sur lequel est soudée la gaine isolante 9.

Le tube 26 est actionnable à l'aide d'une tirette 28 solidaire du tube 26.

En 29 est représenté un joint racleur logé dans une chambre 30 ménagée à l'extrémité du manchon 27 et en 31 est représenté un joint torique interposé entre le tube 26 et la tirette 28 et susceptible de coulisser dans la chambre 30. Un tel dispositif garantit l'étanchéité du pôle auxiliaire lorsque la tirette 28 est en position enfoncée (figure 7) d'obturation de la chambre 30.

La figure 8 représente la tirette 28 en position sortie exposant ainsi une partie du tube 26 sur laquelle peut être fixée la pince crocodile 12 de la figure 5.

Comme il a été vu précédemment, selon la figure 4, la sortie A est à proximité immédiate de l'embout 2 de connexion de la sonde 1 au boîtier de stimulateur, la zone de dérivation étant recouverte d'un manchon isolant 6.

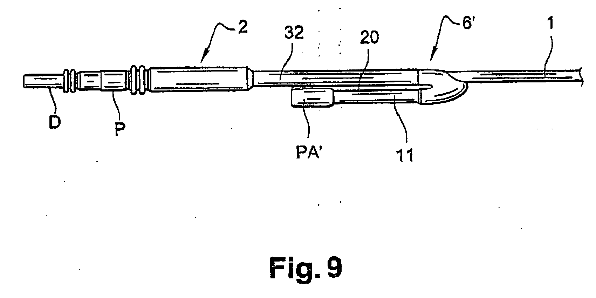

La figure 9 correspond à une variante de positionnement de la sonde auxiliaire pour laquelle la sortie A' est éloignée d'une distance I1 de l'embout 2 de connexion de la sonde 1 par un brin 32, la zone de dérivation étant recouverte d'un manchon isolant 6' pourvu d'un repliement adapté pour appliquer la dérivation 11 le long du brin 32 de sorte que la dérivation reste aisément accessible pour une intervention ultérieure de changement du boîtier, le praticien n'ayant ainsi qu'à suivre le brin 32 à partir du boîter pour dégager le pôle auxiliaire Pa' et procéder à la connexion.

Pour encore mieux maintenir la dérivation 11, un film de colle biocompatible 20 peut avantageusement être utilisé pour solidariser le brin 32 et la dérivation 11.

L'invention ne se limite pas à l'exemple représenté et est notamment applicable à une sonde pour laquelle un adaptateur est intercalé soit entre l'embout 2 et le boîtier de stimulateur soit entre la sonde 1 et les électrodes proximale et distale implantées.

## Revendications

1. Sonde de stimulation cardiaque bipolaire à conducteurs à enroulements colinéaires et à pôle auxiliaire, **caractérisée en ce que** ledit pôle auxiliaire (P_{A}) est relié à l'un des deux connecteurs (D,P) de liaison de la sonde (1) au boîtier de stimulateur par un troisième conducteur à enroulement colinéaire avec les deux conducteurs de la sonde sur un tronçon commun à trois conducteurs entre l'un desdits connecteurs (D,P) et une sortie de dérivation (A, A').

2. Sonde selon la revendication 1, **caractérisée en ce que** ledit conducteur auxiliaire est connecté au connecteur de sonde (D) relié à l'électrode de stimulation distale (E_{D}).

3. Sonde selon la revendication 1 ou 2, **caractérisée en ce que** ledit pôle auxiliaire (P_{A}) est reliée à une sortie (A) du conducteur auxiliaire via un conducteur spiralé dénudé (7) gainé d'un isolant (9).

4. Sonde selon la revendication 3, **caractérisée en ce que** ladite sortie (A) est à proximité immédiate de l'embout (2) de connexion de la sonde (1) au boîtier de stimulateur, la zone de dérivation étant recouverte d'un manchon isolant (6).

5. Sonde selon la revendication 3, **caractérisée en ce que** la sortie (A') est éloignée d'une distance (I1) de l'embout (2), de connexion de la sonde (1) au boîtier, par un brin (32) la zone de dérivation étant recouverte d'un manchon isolant (6') pourvu d'un repliement adapté pour appliquer la dérivation (11) le long du brin (32).

6. Sonde selon la revendication 5, **caractérisée en ce qu'**un film de colle biocompatible (20) solidarise le brin (32) et la dérivation (11).

7. Sonde selon l'une des revendications 4 à 6, **caractérisée en ce que** ledit pôle auxiliaire (P_{A}) est constitué d'un manchon (14) en matériau isolant électrique comportant, disposés coaxialement audit conducteur spiralé (7) et dans son prolongement, deux logements (15,17) de réception d'obturateurs (16,18) en silicone ou analogue, séparés par un conduit axial (19), de diamètre réduit, de guidage d'un stylet (10) ou analogue de liaison électrique dudit conducteur (7) à une source de stimulation auxiliaire, la mise en contact avec le conducteur (7) s'effectuant par insertion du stylet (10) à l'intérieur du conducteur (7).

8. Sonde selon l'une des revendications 4 à 6, **caractérisée en ce que** ledit pôle auxiliaire (P_{A}) est constitué d'un tube conducteur (26) mû par une tirette (28) et coulissant relativement à un fil conducteur (25) relié audit conducteur spiralé (7), ledit tube étant lui-même monté coulissant dans un manchon isolant (27) en sorte de rendre accessible de l'extérieur une partie dudit tube (26) aux fins de connexion à une source de stimulation externe.

9. Sonde selon la revendication 8, **caractérisée en ce qu'**en position enfoncée de la tirette (28) ledit tube (26) est isolé de l'extérieur par un dispositif d'étanchéité (31) interposé entre la tirette (28) et ledit manchon (27) dans lequel coulisse le tube (26).
